# EUROPEAN PATENT APPLICATION

(11) **EP 0 619 992 A1**
(43) Date of publication of application: **19.10.1994**
(21) Application number: 94200659.4
(22) Date of filing: 15.03.1994
(51) Int. Cl.: A61F 9/00, B23K 26/06, G02B 7/00

(54) **Support/positioning device for optical filters**

(30) Priority: 17.03.1993 IT MI930509
(71) Applicant: FONDAZIONE CENTRO SAN ROMANELLO DEL MONTE TABOR, 37031 Illasi (Verona) (IT)
(72) Inventor: Brancato, Rosario, IT-20122 MILANO (IT); Carones, Francesco, IT-20123 MILANO (IT); Gobbi, Pier Giorgio, IT-27100 PAVIA (IT)
(74) Representative: Fusina, Gerolamo

(57) **Abstract**

The support and positioning device for optical filters is capable of varying the spacial intensity distribution of a laser beam emitted by a laser source and striking a surface to be irradiated.

The device (15) is fastened to the base structure (16) of the laser source (10) and comprises several means for translating and tilting said device in space, relatively to an axis of the laser beam emitted by the laser source. The tilting means are constituted by an element bearing an optical filter (13) or "mask", which can be tilted at any angles relatively to the laser beam (11), and can be positioned along each direction in space, spaced apart from a surface to be irradiated, such as a cornea (12).

## Description

The present invention relates to a support/positioning device for optical filters, the so-said "masks", used during eye cornea remodeling surgery.

The technique of use of laser light in order to erode a selected surface of various mechanical pieces, in particular made of plastics materials, has been long known.

In view of these features, the use has widely spread of laser light also in medical sector, in which several biological materials exist which are susceptible of being submitted to ablation treatments by laser light. These biological materials or tissues are represented by cartilages, membranes, ligaments, bones, teeth and, in particular, human eye cornea.

In fact, the latter has been submitted for long time to surgery aiming at modifying the geometrical curvature and hence the refractive properties, in order to correct the most important refractive defects of human eye, such as myopia, hyperopia and astigmatism.

In various cases, such operations are still being carried out according to traditional microsurgical techniques, using surgical instruments for cutting, removing and suturing biological materials.

However, these traditional methods, as already said above, have been overcome long ago by laser surgery, which secures a full range of advantages, which can be found in the extremely high degree of surgical control of ablation, which may even be of less than 1 µm of thickness removed per each laser pulse. Another advantage is the fact that the side thermal damage due to heat conduction is very low and can be observed within the region of from 0.3 to 1 µm.

Argon-fluorine laser, ArF, has been found to be the optimal source for this type of applications, i.e., for surgery interesting human eye cornea. Owing their features, these lasers are used in two different procedures for cornea remodeling.

In the first case, the laser beam is of a size which may be either larger than, or the same as, of the optical area to be treated; on the contrary, in the second case, the beam is smaller than it, and therefore it must be electro-optically deflected in order to allow it to completely treat said area. As to the first method, which secures the homogeneity of the ablation, it surely is more widespread, as well as faster and simpler, than the second one cited above. Unfortunately, it causes several difficulties when geometrically asymmetrical or axially depressed corrections have to be carried out.

This drawback was overcome with the technique disclosed in United States patent Nos. 4,856,513 and 5,019,074. The solution proposed in those documents is of using space filters of pass/not pass type interposed through the optical path of the laser beam, and into contact with the bulb of the eye.

Such filters, so-said "masks", are given the shape of disks or lenses and are composed by materials displaying very similar ablation characteristics to those of cornea, at the same laser wave length. The material of the mask, generally polymers, is subject to a progressive erosion by the laser pulses. The mask consequently starts becoming thinner and thinner, uniformly throughout its transversal thickness. If the thickness of the polymer from which said mask is made, is not constant throughout the cross section of the laser beam, said mask, as erosion advances, Locally turns into transparent to laser light, which can hence reach the underlying cornea. In that way, the mask functions as a space metering device for metering the laser light which strikes cornea, with pass/not pass transmission characteristics which vary during the exposure time. By suitably designing the profile of the mask, a variety of refractive defects can be corrected.

The use of the latter method consists in the substantial reduction in residual roughness of the ablated surface, which allows a faster and more natural healing of corneal epithelium.

A substantial drawback in the art, i.e., in the prior method, is the difficulty of retaining the relative alignment of the laser beam on the mask and of the mask on the cornea.

According to the method and apparatus disclosed in the above said patents, the mask should always be into contact with the patient's head, i.e., his eye. Thus, in order to be capable of aligning the mask, the surgeon should manually control at least four degrees of freedom on the mask, which are two translations on a perpendicular plane, and two inclinations relatively to the axis of the laser beam, while he is performing an operation. Obviously, operating in this way becomes very difficult owing to the high accuracy required by the surgery.

The main purpose of the present invention is of obviating the above exposed drawbacks of the laser equipment, in particular as regards positioning, i.e., adjusting the position of, the masks used during the operation of human cornea remodeling.

Another purpose, connected with the above cited purpose, is of providing a support means for optical filters. masks, which are simple and easy to use, and allow the alignment operations to be simplified, in order to enable the surgeon to correctly operate cornea.

On considering these purposes, a device for supporting and positioning optical filters suitable for varying the space distribution of the intensity of a laser beam emitted by a laser source and striking a surface submitted to irradiation was provided, which is characterized in that said device is fastened to a base structure of said laser source and comprises means for translating and tilting said optical filters in space relatively to the axis of said laser beam, said translation and tilting means being constituted by a carrier element for said optical filters, which is suitable for being tilted according to any angles relatively to said laser beam and can be placed along each direction in space, spaced apart from said surface to be irradiated.

The structural and functional characteristics and the advantages of a device according to the present invention will be better understood by the exemplifying, non-limitative disclosure thereof made by referring to the accompanying drawings, in which:
Figure 1 shows a block diagram of an embodiment of an apparatus for remodeling the contour of a surface, provided with a support/positioning device for an optical filter according to the present invention;
Figure 2 shows a perspective view of the first embodiment of a support/positioning device coupled with a base structure of the laser source, in an operative position;
Figure 3 shows a further perspective view of the structure of Figure 2, in which the support/positioning device is in a rest position:
Figure 4 shows a perspective view of the second embodiment of the support/positioning device, also coupled with a base structure of said laser source.

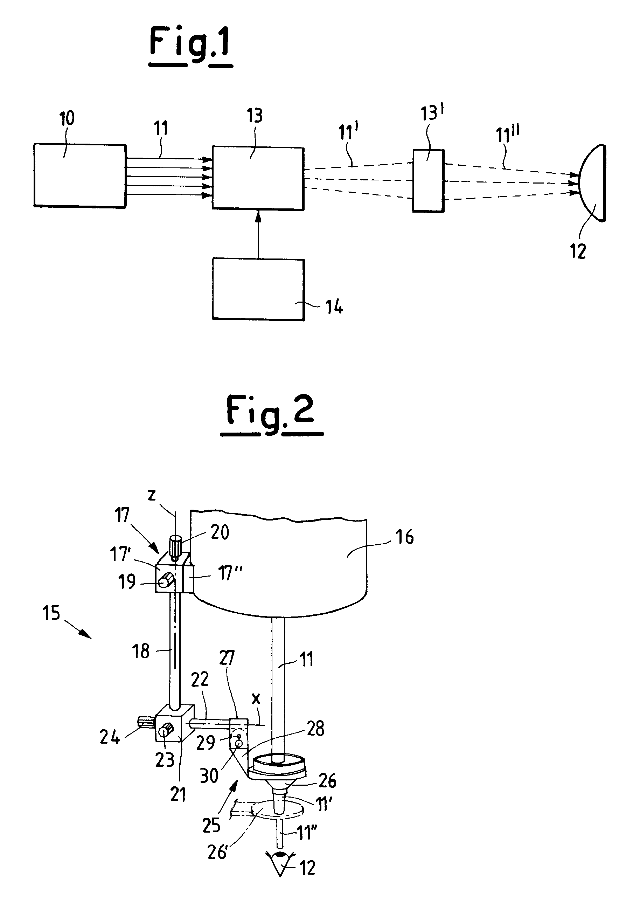

In Figure 1, the block diagram is displayed of an embodiment of an apparatus for remodeling the contour of a surface. A laser light source is schematically shown as a block 10. A laser beam 11, emitted by said source, typically has a circular or square cross section and preferably is such as to completely cover the area of a surface 12 to be remodeled. The intensity of the laser beam 11 preferably is uniform throughout a cross section thereof, at least as regards the irradiated surface 12.

Between the laser source, i.e., the block 10 and the irradiated surface 12 which is the target, an optical device is interposed, i.e., a suitable filter 13 for modifying the space distribution of the laser beam 11 through the cross section of the later. The filter 13, so-said "mask", modifies the beam 11 to yield a beam 11' according to a predetermined pattern, either constantly or in variable fashion during the irradiation time.

As regards the adjustment in position of the optical filter 13, and of a possible further optical device 13', said adjustment in position is carried out by means of a suitable support/positioning device according to the present invention, displayed as a block 14.

Furthermore, the use of the further optical device 13' which is interposed between the optical device 13 and the surface to be irradiated 12, obviously depends on the nature of the individual surgery. Said device, i.e., the optical device 13', produces, on said target surface 12, an image of the beam 11' emerging from the first optical device 13, in the form of a further modification of the beam 11', yielding a beam 11". This embodiment, with the further optical device 13', aims at annihilating any possible damaging effects caused by diffraction and offering the possibility of operating at a longer distance from the target surface 12, besides introducing a possible magnification or reduction factor between the several planes of the laser beam 11, 11' and 11" along the path of the latter.

Turning to Figure 2, a first embodiment of the support and positiong device is shown in greater detail. Said support/positioning device, generally indicated with 15, is represented by the block 14 in Figure 1. The device 15, which is constituted by several means for translation and tilting, is constrained to the base structure 16 of the laser source by means of a suitable fastening device 17, locked, or fastened, onto an outer wall of said base structure 16.

As one may see in Figure 2, the translation and tilting means are constituted by at least two rods perpendicularly constrained to each other and a carrier element 25. In fact, these means enable any inclinations to be obtained of the whole device 15, or of a portion thereof only, relatively to the laser beam. They can furthermore be realized by means of two sliding seats for said rods, which are constituted by bores provided in a support element 21, with which clamps are associated for adjusting and locking the rods.

According to another embodiment, the means for tilting and rotating the device 15 can be constituted by gear wheels and worm screws which are additionally equipped with position fastening elements.

To the fastening device 17 a first rod 18 parallel to the axis of the laser beam 11 and adjustable in height is constrained at a first end thereof by means of a worm screw pin 19, or a sliding seat. Besides being translatable along its own axis z, the rod 18 is also rotatable around it, thanks to a further worm screw 20.

A second end of the first rod 18 is locked to a carrier means 21, to which a second rod 22 is additionally constrained at a first, free end thereof. Said rod 22 is mounted on the carrier 21 in such a way as to be in a perpendicular position to the first rod 18.

Also the second rod 22 is translatable along its own axis x and rotatable around it. In order to perform these movements, on the carrier element 21 two sliding seats for two worm screws are provided, wherein a first worm screw 23 is for performing the translation and the second worm screw 24 is for revolving the rod 22. Of course, also in this case, the seats are furthermore provided with position locking elements.

The second, free end of the second rod 22 is linked, or constrained, to a bearing element 25, the geometry of which can freely vary with the degree of difficulty of the surgical intervention to be carried out. preferably, the carrier element 25 for a filter element 26 is so realized as to allow further inclination angles to be obtained additionaly to those which can be attained with said two rods 18 and 22.

As one will also see in Figure 2, such a carrier element 25 can have an "L"-shaped longitudinal section and consists of two portions 27 and 28, which can be constrained to each other by means of a pin 29 and are fastenable by means of a fastening element 30 which enables an adjustable-friction hinging to be accomplished, and therefore constitute the elements for position adjustment and fixing. The first portion 27 of the carrier element 25 is axially constrained onto the rod 22, and the second portion 28 is rotatable, by a certain angle, around the pin 29.

On either of said two rods 18 and 22 of the device, also a further, freely adjustable in position, optical device 26' -- schematically shown in phantom line -- can be further constrained. In its operating position, the device 26' is placed under the filter element 26 in order to enable the laser beam 11' to be transmitted from said filter element to the surface to be irradiated.

By acting on the hingings, which allow the rods and the carrier element to translate and rotate, the operator can arrange the filter in any desired positions relatively to the laser beam. Owing to this reason, the translation and tilting means can also be equipped with further worm screws and return springs for rods 18 and 22 sliding.

All these elements are provided with fastening elements for locking the position of the device 15, and allow it to be positioned still more precisely.

The filter element 26, resting on the carrier element 25, can be an optical, mechanical or electromechanical device. Such an optical filter, the so-said mask, should be capable of altering the energy distribution of the transmitted laser beam 11' relatively to the entering beam 11, i.e., the beam emitted by the laser beam, before it strikes the working surface 12, in the instant example, a human subject's cornea.

However, the masks can be realized according to various types, their function of intercepting the laser beam and of spatially distributing the transversal intensity in accordance to a pre-established profile, being given for granted. In particular, such a filter can be realized as an absorbing filter, as an erodible mask, or still other. In each of these different realization cases, the support/positioning device 15 according to the present invention enables the mask 26 to be trued on the beam 11 with the desired extreme accuracy, and the desired position to be stably maintained during the course of the operation.

In order to secure the stable positioning of both the mask 26 -- as well as of the possibly installed further optical device 26' schematically shown in phantom line -- and of the whole device 15, all of the mechanisms provided for the adjustments in position, as well as for tilting, can be provided with clamping elements or devices, which enable the adjustable-friction hingings to be accomplished.

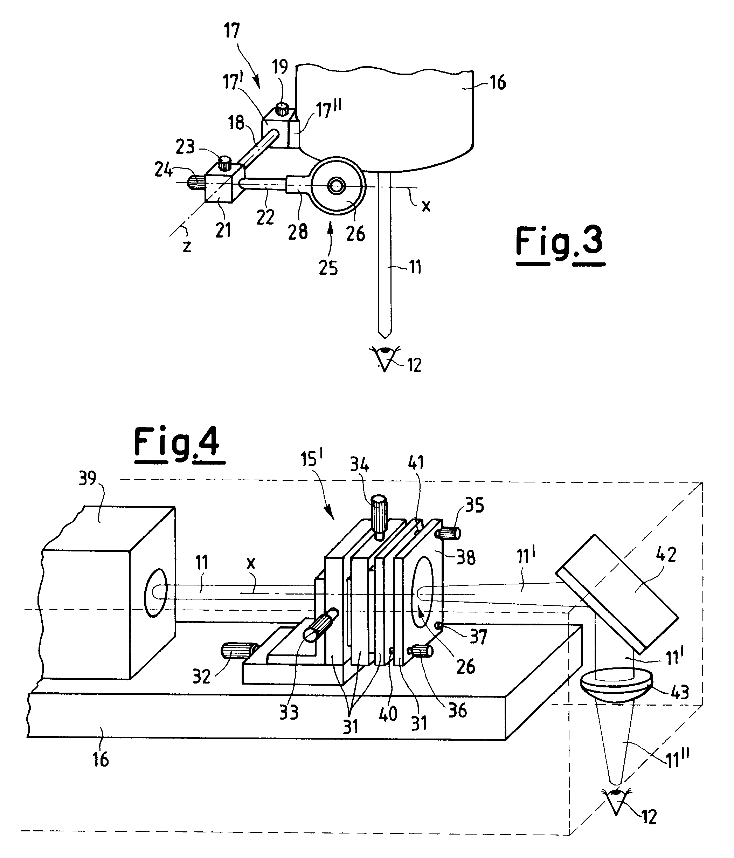

Referring to Figure 3, the whole apparatus is shown, i.e., the base structure 16 of the laser source, and the support/positioning device 15, which is in a rest position. In this position, the whole positioning device 15 is removed away from the laser beam 11, i .e., from its operating position, so the mask 26 does not represent any longer an obstacle for the laser beam 11, which reaches the target surface 12 without being modified.

Reaching its rest position by the device 15 is very simple, and can be accomplished in either of two ways.

In a first case, i.e., as displayed in the Figure, the fastening device 17 is made as two portions 17' and 17", mutually constrained by a friction hinging, not diplayed in Figure. Such a hinging enables the portion 17' to freely rotate around it, i.e., around the portion 17" which is fastened to the base structure 16 of the laser source. In fact, in order to move the support device 15 to its so-said rest position, it is enough that the operator rotates the portion 17' to which the first rod 18 -- and therefore the remainder of the structure of the support device 15 -- is constrained.

In a second case, on the contrary, it is enough that the first rod 18 is slid around its own axis z. In that way, around the same axis the support 21 is rotated, to which, besides the first rod 18, also the second rod 22 -- and, consequently, the bearing element 25 with the mask 26 -- is fastened.

In the case when to the support device 15 also the further optical device 26' is fastened, also the latter reaches its rest position in the same way, witout that the function of the same device is endangered.

Referring to Figure 4, a second embodiment is displayed of the support/positioning device according to the present invention. In this case, the optical filter 26, i.e., the mask, and a support/positioning device 15' can be made integral with a fastening element inside the base structure 16 of the laser source and, consequently, also with other control and viewing apparatuses. The device 15' is fastened to the support by means of a slide with a stop element, or by means of trueing dowels or balls acting by means of a a magnetic attraction mechanism. The translation means can also be constituted by the same frames configured as translating slides running inside purposely provided tracks. However, in any case, the device 15' results to be freely rotatable around its own axis, i.e., around the axis of the laser beam 11.

The support/positioning device 15' is accomplished as a set of frames 31 hinged and articulatedly linked relatively to each other and suitable for housing, or carrying, the optical tool, i.e., the mask 26. Also in this case, the structure of the support/positioning device 15' is so realized as to enable it to be translated in three different directions, and to secure that the same device may reach any inclinations relatively to the laser beam 11, i.e., relatively to the x axis thereof.

The translation movements of the device 15' according to various directions are obtained with the aid of translation means or mechanisms 32, 33 and 34. In fact, by acting on the mechanism 32, the operator will shift the support device 15' along a plane which results to be parallel to the x axis of the laser beam 11. On the contrary, the other mechanisms 33 and 34 make it possible two translation movements to be performed which are perpendicular to each other, on a plane which is perpendicular to the x axis of said beam 11.

In order to enable the device 15', i.e., the optical filter 26, to be tilted at any desired angles to the axis of the laser beam 11, three further threaded pins 35, 36 and 37 are provided. As one may observe in said Figure, the threaded pins 35 and 36 are hinged at two diametrically opposite positions on the frame 38, which is the farthest frame from a laser source 39 provided inside the structure 16; also this frame is a carrier element for an optical tool. Both pins 35 and 36 interact with return springs 40 and 41 and, in combination with the stationary pin 37, make it possible the orientation of both the device 15' and the mask 26 to be changed.

The laser beam 11' modified by the optical filter 26 is additionally reflected inside the base structure 16 of the laser source, by a mirror 42 and is sent towards the second optical device; in this case, a lens 43. In fact, the latter transmits to the target surface 12, i.e., the eye cornea, an enlarged or reduced image of the beam 11' emerging from the first optical tool 26.

Also in this case, as, e.g., in the case of the first embodiment of the support device, the translation/tilting means and the rotating means in space, can be provided with clamping means which make it possible the adjustable-friction hingings to be accomplished. In order to be able to secure a stable positioning of the device during the surgery, such means are equipped with elements for position fixing.

In both of the embodiments displayed in Figures 2 and 4, the optical filter 26 can be freely rotated inside the bearing element 25, which can be further equipped with elements for fixing the position of said optical filter 26.

The primary advantage of the device according to the present invention for supporting and positioning optical filters according to both the first and the second embodiments, is the position of the optical filter during the surgery. In fact, the mask is no longer into direct contact with the patient's eye, to the contrary, is placed rather spaced apart from it. In this way, the surgeon is advantageously given the possibility of operating at a longer distance from the surface; in the specific case discussed herein, from eye.

Another advantage of the device according to the present invention is offered by the possibility of performing the translational movements according to any possible directions, and furthermore obtaining any inclinations relatively to the axis of the laser beam.

A further advantage, also secured by the structure of the device, is offered by the simpleness with which the above said movements can be carried out with no risks for the outcome of the surgery. In fact, the support/positioning device makes it possible a stable mutual alignment to be accomplished of the optical tool and the laser beam, and of the optical tool and the surface to be remodeled.

Not least advantage also is the easy and simple use of the carrier device, which requires very simple operations for the adjustment in position, to the benefit of the correctness of function. The advantage results hence obvious of the support/positioning device according to the present invention, which allows the several translational and tilting movements to be performed, which are necessary in order to position the filter, which may be of a whatever type, in any desired positions relatively to the laser beam.

## Claims

1. Device for supporting and positioning optical filters suitable for varying the space distribution of the intensity of a laser beam emitted by a laser source and striking a surface submitted to irradiation, characterized in that said device is fastened to a base structure of said laser source and comprises means for translating and tilting said optical filters in space relatively to the axis of said laser beam, said translation and tilting means being constituted by a carrier element for said optical filters, which is suitable for being tilted according to any angles relatively to said laser beam and can be placed along each direction in space, spaced apart from said surface to be irradiated.

2. Device according to claim 1, characterized in that said translation means and said tilting means are constituted by at least two mutually perpendicular rods, wherein a first rod of said two rods is further constrained, at a first end thereof, to a fastening element fastened, in its turn, onto said base structure of said laser source, and a second rod of said two rods bearing said bearing element at a free end thereof.

3. Device according to claim 2, characterized in that said at least two rods are constrained to each other by means of a support element.

4. Device according to claim 2, characterized in that said bearing element is constituted by two portions pivotally hinged to each other, with said portions being provided with position adjustment and fixing means.

5. Device according to claim 4, characterized in that one of said two portions of the bearing element is suitable for being fastened onto said free end of said second rod.

6. Device according to claim 2, characterized in that said tilting means are additionally constituted by sliding seats for both said rods, with adjustment and fastening clamps.

7. Device according to claim 2, characterized in that said tilting means are constituted by gear wheel and screws, with said means being equipped with position fixing means.

8. Device according to claim 2, characterized in that said fastening element is constituted by two portions hinged to each other, with a first portion of said two portions being hinged onto said base structure of said laser source and a second rod being rotatable around said first fixed portion.

9. Device according to claim 2, characterized in that said translation means are additionally constituted by sliding seats for said two rods with adjustment clamps.

10. Device according to claim 2, characterized in that said translation means include worm screws and return springs for causing said at least two rods to slide, with said means being provided with position fixing elements.

11. Device according to claim 1, characterized in that said translation means and tilting means are constituted by a set of frames articulatedly linked to each other and suitable for being integrally fastened to a fastening element inside said base structure of said laser source.

12. Device according to claim 11, characterized in that at least one from said set of frames is a carrier element.

13. Device according to claim 11, characterized in that said carrier element is equipped with elements for rotating and fixing the position of said optical filters.

14. Device according to claim 11, characterized in that said translation means are constituted by frames shaped as sliding guides translatable inside relevant guides.

15. Device according to claim 14, characterized in that said sliding guides are provided with screws and return springs.

16. Device according to claim 11, characterized in that said tilting means are constituted by hinged frames equipped with springs.

17. Device according to claim 11, characterized in that it comprises a mirror which can be positioned between the optical filter and the second optical device.

18. Device according to claim 11, characterized in that the second optical device is a lens.

19. Device according to claim 11, characterized in that said fastening element is constituted by trueing-dowel elements.

20. Device according to claim 11, characterized in that said fastening element is constituted by magnetic-attraction balls.

21. Device according to claim 2 or 11, characterized in that it additionally comprises a second optical device suitable for transmitting said laser beam from said optical filters to said surface to be irradiated.

22. Device according to claim 2 or 11, characterized in that said optical filter is freely rotatable inside said carrier element.

23. Device according to any of preceding claims, characterized in that said optical filter is an absorbing filter.

24. Device according to any of preceding claims, characterized in that said optical filter is a reflecting mirror.

25. Device according to any of the preceding claims, characterized in that said mask is an erodible mask.

26. Device according to any of preceding claims, characterized in that said surface to be irradiated is a biological tissue.

27. Device according to any of preceding claims, characterized in that said surface to be irradiated is corneal tissue.
